Europäisches Patentamt

(19) European Patent Office

Office européen des brevets

(11) Veröffentlichungsnummer: **0 081 122**
B1

# (12) EUROPÄISCHE PATENTSCHRIFT

(45) Veröffentlichungstag der Patentschrift:
02.10.85

(21) Anmeldenummer: 82110701.8

(22) Anmeldetag: 19.11.82

(51) Int. Cl.⁴: **C 07 K  5/02**, A 61 K  37/02

(54) **Carrier Tripeptid mit antifungischer Wirksamkeit.**

(30) Priorität: 08.09.82 DE 3233679

(43) Veröffentlichungstag der Anmeldung:
15.06.83 Patentblatt 83/24

(45) Bekanntmachung des Hinweises auf die Patenterteilung:
02.10.85 Patentblatt 85/40

(84) Benannte Vertragsstaaten:
AT BE CH DE FR GB IT LI LU NL SE

(56) Entgegenhaltungen:
CHEMICAL ABSTRACTS, Band 83, Nr. 21, 24. November 1975, Seite 14, Nr. 172408u, Columbus, Ohio, USA K. EISELE: "Sequence variation on an antibiotically active tripeptide"
EXPERIENTIA, Band 31, Fasc. 7, 15. Juli 1975, Seiten 764-765, Basel, CH., K. EISELE: "Antibiotisch wirksame tripeptide der sequenz L-Arg-D-X-L-Phe"
CHEMICAL ABSTRACTS, Band 83, Nr. 23, 8. Dezember 1975, Seite 506, Nr. 193691w, Columbus, Ohio, USA, A. EISELE et al.: "Antibiotically active tripeptides with the sequence
l-arginine-N-alpha-methyl-L-X-L-phenylalanine"

(73) Patentinhaber: Helopharm W. Petrik & Co.KG.,
Waldstrasse 23-25, D-1000 Berlin 51 (DE)

(72) Erfinder: Meyer-Glauner, Wilhelm, Dr.med., Albweg 8,
D-7400 Tübingen (DE)
Erfinder: Echner, Hartmut, Dipl. Chem., Bachgasse 15,
D-7400 Tübingen (DE)

(74) Vertreter: Miehe, Manfred, Dipl.-Chem., Falkenried 4,
D-1000 Berlin 33 (DE)

## Beschreibung

Es ist bekannt, daß Oligopeptide physiologische Wirksamkeit besitzen. So weisen das Oxytocin und Vasopressin als Hinterlappenhormone neun Aminosäurereste auf. Das Phalloidin als Giftstoff des Knollenblätterpilzes weist sieben Aminosäurereste auf.

Es ist aber auch bekannt, daß relativ niedermolekulare Oligopeptide z. B. bei der Zellatmung wichtige physiologische Funktionen ausüben. So bewirkt das Tripeptid Glutathion, das das L-Glutaminyl-L-cysteinyl-L-glycin ist, die Funktion eines intermediären Wassertoffüberträgers in der Atmungskette im Zusammenhang mit der ATP-Bildung auf, und zwar aufgrund der reversiblen Oxidation der HS-Gruppe des Cysteinbausteins.

Es gibt zahlreiche biochemische Antagonisten oder Antimetaboliten, deren Wirkungsmechanismus teilweise noch ungeklärt ist. Ein bekanntes einfaches Beispiel stellt die vor etwa 50 Jahren eingeführte Sulfonamidtherapie dar, die das erste Beispiel in der Medizin ist für das Anwenden eines Antagonisten, der ein Strukturanaloges der p-Aminobenzoesäure ist, die für die bakterielle Folsäurebiosynthese erforderlich ist, so daß die Mikroorganismen aufgrund des Einbaues einer falschen Verbindung zum Absterben gebracht werden.

Es ist weiterhin bekannt geworden, daß es auf dem Gebiet der niederen Oligopeptide Antimetaboliten geben kann. So wurde festgestellt, daß das durch Keratinophyton terreum gebildete L-Arginyl-D-allothreonyl-L-phenylalanin eine antifungische Wirksamkeit besitzt. (Synthese, biologische Aktivität und medizinische Bedeutung von Peptiden der Sequenz L-Arg-X-L-phe. Universität Tübingen, Dissertation 1975). Im Hinblick auf die ungewöhnliche D-Konfiguration des mittelständigen Aminosäurerestes sind Tripeptide synthetisiert worden, die in der Mittelstellung N-$\alpha$-Methyl oder D-Aminosäurereste aufweisen. Es wurde hierbei gefunden, daß von den untersuchten Tripeptiden L-Arginyl-D-phenylalanyl-L-phenylalanin und L-Arginyl-N-$\alpha$-methyl-L-phenylalanyl-L-phenylalanin bezüglich ihrer biologischen Wirksamkeiten der oben genannten natürlich vorkommenden Verbindung am nächsten kommen. Diese Peptide zeigen jedoch gegenüber Problemkeimen wie insbesondere candida albicans keinerlei oder nur eine sehr schwache wachstumshemmende Aktivität.

Gemäß der Literaturstelle »Experientia« Band 31, Fasc. 7, (1975) Seiten 764—765 sind die nachfolgenden Peptide bekannt:

L-Arg-D-Ala-L-Phe, L-Arg-D-Tyr-L-Phe, L-Arg-D-Phe-L-Phe,
L-Arg-D-Le-L-Phe, L-Arg-D-Val-L-Phe.

Es ist hier angegeben, daß diese Tripeptide neben Paecilomyces varioti (einem Ascomyceten) und Mucor miehei (einem Zygomyceten) auch die vor allem klinisch und von der Pathogenität her wichtigen Deuteromyceten Candida albicans, candida pseudotropicalis, Gastricum candidum, Nematospora corglii und Tomlopsis glasrate hemmen. Es sind allerdings keinerlei minimale Hemmkonzentrationen angegeben, was bei einer so wichtigen Befunderhebung absolut notwendig ist und sind auch späterhin nicht von dem Verfasser nachveröffentlicht worden.

Untersuchungen mit diesen fünf Tripeptiden, hergestellt nach der Merrifield-Fest-körper-Methode und nach der von dem Verfasser benutzten Testmethodik (Filterrondellen-Test auf homogener Keimschicht) zeigten, daß gegenüber den angegebenen Testorganismen bei Konzentrationen bis zu 10 mg/ml keinerlei Wachstumshemmungen in Form von Hemmzonen um die mit den Peptiden getränkten Filterrondellen festgestellt wurden.

Die weiteren Untersuchungen dieser fünf Tripeptide gegenüber Cadida albicans und Candida pseudotropicalis in »yeast nitrogen base« Medium mit einer Zellzahl von $10^4$/ml zu Testbeginn führten zu dem Ergebnis, daß diese Tripeptide keinerlei Wachstumshemmung zeigen. Die Tripeptide werden dabei bis zu einer Konzentration von 10 mg/ml Testmedium beurteilt. Bei den Tripeptiden L-Arg-D-Val-L-Phe und L-Arg-D-Ala-L-Phe werden im Gegenteil sogar Wachstumsförderung von Candida albicans festgestellt.

Demgegenüber liegt der Erfindung nun die Aufgabe zugrunde, durch weitere molekulare Veränderungen des mittelständigen Bausteins im nachfolgenden X genannt, der eingangs genannten Verbindung L-Arginyl-X-L-phenylalanin, Verbindungen herzustellen, die bestimmte zweckmäßige physiologische Eigenschaften aufweisen.

Diese Aufgabe wird erfindungsgemäß dadurch gelöst, daß die folgenden Tripeptide

L-Arginyl-DL(p-fluorphenylalanyl)-L-phenylanilin,
L-Arginyl-DL-(m-fluortyrosyl)-L-phenylalanin,
N$^1$-Acetyl-L-arginyl-DL-(p-fluorphenylalanyl)-L-phenylalanin und
L-Arginyl-DL-(p-fluorphenylalanyl)-L-phenylalaninamid
geschaffen werden.

Es wurde überraschenderweise gefunden, daß die Verbindungen 1 und 2 nicht nur eine vergleichbare biologische Aktivität wie die natürlich vorkommende Verbindung L-Arg-D-allothre-L-phe besitzen, sondern darüber hinaus auch sehr gute Wirksamkeit gegen insbesondere candida albicans aufweisen.

Der Erfindungsgegenstand wird nachfolgend beispielsweise erläutert.

## Beispiel 1

Die Herstellung der Verbindungen erfolgte aus den drei Komponenten vermittels des Merrifield Verfahrens (Feststoffphase-Verfahren). Hierbei erfolgt zunächst eine Chlormethylierung eines Copolymeren aus 98% Styrol und 2% Divinylbenzol, sodann Anesterung der N-t-BOC-Aminosäure an das Trägerharz in Form des genannten Copolymer, Anwenden von DCCl als Kupplungsreagens und sodann Ablösen des fertigen, geschützten Peptids als freie Säure mit Bromwasserstoffsäure und Trifluoressigsäure oder Eisessig oder mit Bor. Die Acetylierung des N-terminalen L-Argininrestes mit Acetanhydrid/Pyridin wird vermittels des Verfahrens von Merrifield ausgeführt. Die $\alpha$-Carboxamidbildung an dem C-terminalen L-Phenylalanin wird vermittels eines Verfahrens durchgeführt, bei dem ein Benzhydrylamin-Harz angewandt wird. Nach der Fluorwasserstoffabspaltung werden die Peptide vermittels Säulenchromatographie mit Silikagel gereinigt. Die Peptide werden bei 4°C mit n-Butanol : $H_2O$ : Essigsäure in einem Verhältnis von 4 : 2 : 1 eluiert. Die bei den antifungischen Untersuchungen angewandten Tripeptide erwiesen sich als homogen vermittels Dünnschichtchromatographie an Silikagel unter Anwenden von drei Lösungsmittelsystemen. Die relativen Aminosäure-Zusammensetzungen werden unter Anwenden eines Beckman 121 Aminosäureanalysators bestätigt.

## Beispiel 2

Die antifungischen Wirksamkeiten werden gegenüber Testisolaten von Paecilomyces varioti und Mucor miehei, candida albicans, candida tropicales, candida pseudotropicales, candida krusei, aspergillus fumatus, aspergillus niger und weiteren auf einem komplexen Medium und Malzextrakt-Agar untersucht.

In der beigefügten Tabelle I sind die kleinsten inhibierenden Konzentrationen der beiden beispielsweisen erfindungsgemäßen Tripeptide 1 und 2 denjenigen der freien Antimetaboliten gegenübergestellt anhand des Beispiels candida albicans. Die Untersuchungen wurden in einem yeast nitrogen base Medium mit einem Glukosegehalt von 2% und einem $p_H$-Wert von 7,2 durchgeführt. Die Zellzahl zu Versuchsbeginn beläuft sich auf $10^4$/ml. Die Tests werden in Mikrotiterschalen durchgeführt und es wird 24 Stunden bei 30°C inkubiert. Der N-terminal acetylierte Abkömmling des Peptids 1, die Verbindung 3 und der C-terminal amidierte Abkömmling des Peptids 1, die Verbindung 4, besitzen eine auf etwa 50% reduzierte Aktivität des Peptids 1, zeichnen sich aber dadurch aus, daß sie durch Carboxypeptidasen schwerer spaltbar sind.

Keines der Peptide zeigt antibakterielle Wirksamkeiten gegenüber Pseudomonas aeruginosa, Escherichia coli, Staphylococcus aureus, Proteus mirabilis, Klebsiella pneumoniae und Bacillus subtilis. Auch hier ist es wiederum von Interesse festzustellen, daß die Aminosäure-Antagonisten p-F-DL-Phenylalanin und $\beta$-2-DL-Thienylalanin, die gegenüber einigen Bakterien Wirksamkeit zeigen, dieselbe dann verlieren, wenn sie in Tripeptiden der angegebenen Formeln vorliegen.

Tabelle I
Minimale Hemmkonzentration bei candida albicans
a) Anzahl der inhibierenden Isolate
b) minimale inhibierende Konzentration = µg/ml (mMole/l)

| | L-Arg-p-F-DL-Phe-L-Phe | p-F-DL-Phe | L-Arg-m-F-DL-Tyr-L-Phe | m-F-DL-Tyr |
|---|---|---|---|---|
| Candida | a) 8 | a) 0 | a) 2 | a) 0 |
| albicans | b) $<32\,(6,5 \times 10^{-5})$ | b) $\leq 250\,(1,4 \times 10^{-3})$ | b) $\leq 125\,(2,5 \times 10^{-4})$ | b) $\leq 250$ $(1,3 \times 10^{-3})$ |
| 10) | a) 2 | | | |
| | b) $<125\,(2,6 \times 10^{-4})$ | | | |

Die Tripeptide 5, 6, 7 und 8 wurden gegenüber candida albicans, candida tropicalis, aspergillus fumigatus und aspergillus flavus getestet bezüglich der minimalen inhibierenden Konzentration $MIC^{50}$ und $MIC^{100}$ und dieselbe mit dem freien Metaboliten verglichen.

Die Versuchsergebnisse sind in den nachfolgenden Tabellen wiedergegeben, die zum einen die

**0 081 122**

minimal inhibierenden Konzentrationen für 50 und 100 für die erfindungsgemäßen Verbindungen, und zum anderen für den freien Metaboliten wiedergeben.

Tabelle II

| Verbin-dung | candida albicans $\text{MIC}^{50}$ | $\text{MIC}^{100}$ | candida tropicalis $\text{MIC}^{50}$ | $\text{MIC}^{100}$ | aspergillus fumigatus $\text{MIC}^{50}$ | $\text{MIC}^{100}$ | aspergillus flavus $\text{MIC}^{50}$ | $\text{MIC}^{100}$ |
|---|---|---|---|---|---|---|---|---|
| 5 | 4 | 8 | 4 | 16 | 2 | 4 | 2 | 32 |
| 6 | 32 | 64 | 4 | 16 | 32 | 32 | 16 | 64 |
| 7 | 32 | 64 | 32 | 64 | 64 | 64 | 32 | 64 |
| 8 | 16 | 32 | 8 | 16 | 4 | 16 | 8 | 16 |

Tabelle III

| Verbindung | candida albicans $\text{MIC}^{50}$ | $\text{MIC}^{100}$ | candida tropicalis $\text{MIC}^{50}$ | $\text{MIC}^{100}$ | aspergillus fumigatus $\text{MIC}^{50}$ | $\text{MIC}^{100}$ | aspergillus flavus $\text{MIC}^{50}$ | $\text{MIC}^{100}$ |
|---|---|---|---|---|---|---|---|---|
| p-J-Phenylalanin | 64 | >128 | | >128 | 16 | 32 | 16 | 64 |
| p-Methoxyphenylalanin | | >128 | | >128 | | >128 | | >128 |
| p-Nitrophenylalanin | | >128 | | >128 | 64 | >128 | 128 | >128 |
| p-Br-phenylalanin | | >128 | | >128 | 16 | 128 | 32 | >128 |

Die tabellarisch zusammengefaßten Versuchsergebnisse zeigen, daß diese erfindungsgemäßen Verbindungen eine hohe antifungische Wirksamkeit gegenüber einer Mehrzahl an Pilzen aufweisen, die von großer klinischer Bedeutung sind.

Die Tabelle III zeigt die relative Unwirksamkeit der freien Metaboliten, die als atypische Aminosäurekomponente in den Tripeptiden aufgrund eines bisher noch nicht vollständig geklärten Mechanismus durch die beiden herkömmlichen, endständigen Aminosäuren in dem zu bekämpfenden Mikroorganismus wirksam werden.

Das Tripeptid 5 ist erstmals wirksam gegen candida tropicalis. Mit der Synthese dieser neuen Verbindungen ist es zum ersten Mal gelungen, in dieser Serie den Pilz trichophyton metagrophytes zu hemmen.

Es werden jeweils 100 mg Tripeptid bzw. freier Metabolit pro Milliliter physiologische Natriumchloridlösung angewandt und in Filterrondellen (Firma Oxoid) eingeführt und sodann getrocknet. Es erfolgt eine Überführung in mit Agar-Nährmedium beschickte Petrischalen. Die Inkubierung wird 36 bis 72 Stunden bei 37°C ausgeführt. Die Versuchsergebnisse sind in der folgenden Tabelle IV angegeben, wobei TM = trichophyton metagrophytes und i- = inkomplett (keine vollständige Wirksamkeit) bedeuten.

Tabelle IV

| Verbindung | Pilz | Hemmzonendurchmesser | freier Metabolit |
|---|---|---|---|
| 5 | TM | 33 | 0 |
| 6 | TM | 20 | 0 |
| 7 | TM | 19i | i |
| 8 | TM | 28 | 16 |

4

Die erfindungsgemäßen Verbindungen zeichnen sich gegenüber den vorbekannten Verbindungen insbesondere dadurch aus, daß ein breiterer Wirkungsbereich gegenüber pathogenen Pilzen gegeben ist. Während p-F-DL-Phenylalanin gegenüber Candida albicans nicht wirksam ist, zeigt sich eine ausgeprägte Wirksamkeit wenn diese Verbindung als X in dem eingangs genannten Tripeptid vorliegt. So werden alle überprüften Candida albicans Isolate durch weniger als 128 µg/ml des Peptids 1 inhibiert, während der freie Aminosäureantagonist keine Inhibierung bei einer Konzentration bis zu 256 µg/ml zeigt. Auf der molaren Grundlage weist dieses Tripeptid somit eine mehr als 20fach größere Wirksamkeit gegenüber 80% der überprüften Isolate auf. Candida krusei, es werden 10 Isolate überprüft, und Cryptococcus neoformans, es werden 4 Isolate überprüft, werden durch keine der erfindungsgemäßen Verbindungen inhibiert.

**Patentanspruch**

Anwendung der Tripeptide

A)  L-Arginyl-{DL-(p-fluorphenylalanyl)}-L-phenylalanin,
B)  L-Arginyl-{DL-(m-fluortyrosyl)}-L-phenylanalin,
C)  N$^1$-Acetyl-L-arginyl-{DL-(p-fluorphenylalanyl)}-L-phenylalanin und
D)  L-Arginyl-{DL-(p-fluorphenylalanyl)}-L-phenylalaninamid

als antifungisches Mittel, insbesondere mit Wirksamkeit gegen candida albicans.

**Claim**

Use of the tripeptides

A)  L-arginyl-{DL-(p-fluorophenylalanyl)}-L-phenylalanine,
B)  L-arginyl-{DL-(m-fluorotyrosyl)}-L-phenylalanine,
C)  N$^1$-acetyl-L-arginyl-{DL-(p-fluorophenylalanyl)}-L-phenylalanine and
D)  L-arginyl-{DL-(p-fluorophenylalanyl)}-L-phenylalanine amide

as antifungal agent, in particular with effectiveness against candida albicans.

**Revendication**

Applications des Tripeptides

A)  L-Arginyl-[DL-(p-fluorophénylalanyl)]-L-phénylalanine
B)  L-Arginyl-[DL-(m-fluorotyrosyl)]-L-phénylalanine
C)  N$^1$-Acétyl-L-arginyl-[DL-(p-fluorophénylalanyl)]-L-phénylalaline et
D)  L-Arginyl-[DL-(p-fluorophénylalanyl)]-L-phénylalaninamide

en tant qu'agents antifongiques, notamment agissant contre les Candida albicans.